# EUROPEAN PATENT APPLICATION

(11) **EP 4 169 530 A1**
(43) Date of publication of application: **26.04.2023**
(21) Application number: 21825363.1
(22) Date of filing: 17.06.2021
(51) Int. Cl.: A61K 39/395, C07K 16/28, A61P 35/00

(54) **STABLE FORMULATION FOR RECOMBINANT ANTI-PD-1 MONOCLONAL ANTIBODY**

(30) Priority: 19.06.2020 CN 202010566153
(71) Applicant: Sinocelltech Ltd., Beijing 100176 (CN)
(72) Inventor: HU, Ping, Beijing 100176 (CN); LIU, Yan, Beijing 100176 (CN); SUN, Chunyun, Beijing 100176 (CN); HUAI, Qingru, Beijing 100176 (CN); TIAN, Shaomei, Beijing 100176 (CN); TAO, Mingzhen, Beijing 100176 (CN)
(74) Representative: Dolphin, Kirsty Mairi
(86) International application number: PCT/CN2021/100658
(87) International publication number: WO 2021/254447

(57) **Abstract**

Provided is a stable formulation for a recombinant monoclonal antibody, consisting of a recombinant anti-PD-1 monoclonal antibody, a buffer, an osmotic pressure regulator, a stabilizer, and a surfactant. The pharmaceutical formulation may enhance the stability of the antibody and prolongs a validity period of the antibody in aqueous formulations.

## Description

### CROSS-REFERENCING OF RELATED APPLICATIONS

This application claims the benefit of Chinese patent application 202010566153.8 filed on June 19, 2020, the contents of which are incorporated herein by reference.

### FIELD

The present invention relates to the field of biopharmaceutical preparations, and in particular to a stable formulation for recombinant anti-PD-1 monoclonal antibodies.

### BACKGROUND

PD-1, known as programmed cell death receptor 1, is a member of the CD28 family, also known as CD279. It functions as a regulator of programmed cell death and is mainly expressed on the surface of mature CD4+ and CD8+ T cells, and also natural killer T cells, B cells, monocytes, and some dendritic cells. PD-1 is involved in the immune regulation of T cells.

PD-1 has two ligands: PD-L1 and PD-L2, which are normally expressed on antigen-presenting cells and, upon binding to PD-1, inhibit the activation and proliferation of T cells. Under normal circumstances, the immune system responds to foreign antigens that accumulate in the lymph nodes or spleen, promoting the antigen specific activation and proliferation of T cells. In contrast, the binding of PD-1 to PD-L1 conducts negative regulatory signals and inhibits T-cell activation and proliferation.

One of the ways for tumor cells to evade T cell killing is expressing PD-L1 on their cell surface. When PD-L1 binds to PD-1 on the surface of tumor antigen-specific T cells, it conducts negative regulatory signals, making tumor antigen-specific T cells unable to monitor tumor cells and to send attack signals to tumor cells, which leads to tumor cells evading immune surveillance and killing by the body.

Antibodies against PD-1 specifically bind PD-1, block its interaction with PD-L1, and disarm PD-1/PD-L1-mediated T-cell immunosuppression, thereby inducing T-cell activation and re-establishing the body's ability to monitor and attack tumor cells.

However, before the administration, antibody formulations undergo storage and transport processes during which physical and chemical degradation of the antibody occurs, and these instabilities may reduce the potency and/or increase the immunogenicity of the antibody, thus a stable formulation to ensure that the antibody remains therapeutically active and safe until the administration is needed.

### SUMMARY

The technical problem to be solved by the present invention is to provide a stable formulation for a recombinant anti-PD-1 monoclonal antibody. The recombinant anti-PD-1 monoclonal antibody has good stability in this formulation.

The first aspect of the invention relates to a stabilized formulation comprising a recombinant anti-PD-1 monoclonal antibody, a buffer, an osmolarity regulator, a stabilizer, and a surfactant.

In one of its embodiments,
the concentration of said recombinant anti-PD-1 monoclonal antibody is 10-50 mg/mL, preferably a recombinant anti-PD-1 monoclonal antibody of 10-25 mg/mL; and
the concentration of said buffer is 10-50 mM, preferably a buffer of 20-40 mM;
the concentration of said osmolality regulator is 20-200 mM, preferably 80-160 mM;
the concentration of said stabilizer is 10-250 mM, preferably 20-205 mM;
the concentration of said surfactant is 0.005-0.05 wt%, preferably 0.02-0.04 wt%;
the pH of the solution is 5.5-6.5, preferably 5.8-6.2.

In one of its embodiments,
said buffer is selected from one or more of citric acid buffer, acetate buffer, or histidine buffer;
said osmolality regulator is selected from sodium chloride;
said stabilizer is one or more of sucrose, trehalose, or arginine hydrochloride, preferably 205 mM sucrose, or 20 mM - 80 mM arginine hydrochloride, or 200 mM trehalose;
said surfactant is selected from polysorbate 80.

In one of its embodiments,
the pH of the formulation solution is 6.0.

In one of its embodiments,
the formulation contains 25 mg/mL recombinant anti-PD-1 monoclonal antibody; 20 mM histidine buffer, 120 mM sodium chloride, 40 mM arginine hydrochloride, and 0.02 wt% polysorbate 80.

In one of its embodiments,
said recombinant anti-PD-1 monoclonal antibody comprises a light chain variable region and/or a heavy chain variable region.
wherein the light chain variable regions comprise a light chain CDR1 with amino acid sequence SEQ ID NO:1, a light chain CDR2 with amino acid sequence SEQ ID NO:2, and a light chain CDR3 with amino acid sequence SEQ ID NO:3; and
the heavy chain variable regions comprise heavy chain CDR1 with amino acid sequence SEQ ID NO:4, heavy chain CDR2 with amino acid sequence SEQ ID NO:5, and heavy chain CDR3 with amino acid sequence SEQ ID NO:6.

In one of its embodiments,
said recombinant anti-PD-1 monoclonal antibody comprises an amino acid sequence having at least 90%, 92%, 95%, 98%, or 100% sequence identity to the PD-1 antibody light chain variable region sequence SEQ ID NO:8, and/or an amino acid sequence having at least 90%, 92%, 95%, 98%, or 100% sequence identity to the PD-1 antibody heavy chain variable region sequence SEQ ID NO:7.

In one of its embodiments,
said antibody further comprises a light chain constant region and a heavy chain constant region, preferably the amino acid sequence of said light chain constant region having at least 90%, 92%, 95%, 98%, or 100% sequence identity to the kappa light chain constant region of SEQ ID NO: 10, and/or the amino acid sequence of said heavy chain constant region having at least 90%, 92%, 95%, 98% or 100% sequence identity to the IgG4 heavy chain constant region of SEQ ID NO:9.

In one of its embodiments,
said recombinant anti-PD-1 monoclonal antibody is an IgG antibody, preferably an IgG4 antibody.

In one of its embodiments,
said recombinant anti-PD-1 monoclonal antibody is a monoclonal antibody.

In one of its embodiments,
The mean KD, binding affinity of said recombinant anti-PD-1 monoclonal antibody to recombinant human PD-1 protein is 20-200 pM, preferably 60-70 pM, more preferably 64.8 pM.

In one of its embodiments,
the formulation is in aqueous form or lyophilized form.

In one of its embodiments,
the formulation can be stored stably for at least 42 months at 2~8°C and at least 12 months at 25°C.

The second aspect of the present invention relates to the use of formulations of the present invention in the preparation of medicaments for the treatment of tumors or cancers, preferably colon cancer.

The third aspect of the present invention relates to the use of the formulations of the present invention for the treatment of tumors or cancers preferably colon cancer.

### BRIEF DESCRIPTION OF THE DRAWINGS

Figure 1 shows the purity trend of each formulation sample in Example 1 at 4°C and 37°C at week 0, week 3, and week 5.
Figure 2 shows the trend of acidic peaks for each formulation sample in Example 1 at 4°C and 37°C for week 0, week 3, and week 5.
Figure 3 shows the purity trend of each formulation sample in Example 2 at 4°C and 37°C for week 0, week 3, and week 5.
Figure 4 shows the trend of acidic peaks for each formulation sample in Example 2 at 4°C and 37°C for week 0, week 3, and week 5.
Figure 5 shows the purity trend of each formulation sample in Example 3 at 4°C and 37°C for week 0, week 3, and week 5.
Figure 6 shows the trend of acidic peaks for each formulation sample in Example 3 at 4°C and 37°C for week 0, week 3, and week 5.
Figure 7 shows the purity trend of each formulation sample in Example 4 at 4°C and 37°C for week 0, week 3, and week 5.
Figure 8 shows the purity trend of each formulation sample in Example 5 at -80°C and 45°C for week 0, week 1, week 2, and week 4.

### DETAILED DESCRIPTION

The present invention provides a stable formulation for a recombinant anti-PD-1 monoclonal antibody that solves the problem of antibody stability during storage and transport. The antibody is guaranteed to remain active and safe for therapeutic purposes before administration to patients.

The term "formulation" refers to a composition that maintains the biological activity of the active component in an effective manner and does not contain other components that are unacceptably toxic to the subject. Such preparations are sterile. The term "sterile" refers to the absence of live bacteria or the absence or substantial absence of all live microorganisms and their spores.

As used herein, a "stable" formulation refers to a formulation in which the active component retains substantially its physical stability and/or chemical stability, and/or biological activity after storage. Preferably, the formulation retains substantially its physical and chemical stability, as well as its biological activity after storage.

The terms "patient" or "subject" are used interchangeably and refer to any mammal suffering from a condition or disease in accordance with the present invention. Preferably, human.

The stabilized formulation of the present invention comprises a recombinant anti-PD-1 monoclonal antibody, a buffer, an osmolarity regulator, a stabilizer, and a surfactant.

The terms "comprise" and "contain" mean that additional components may be included in addition to those mentioned.

When used herein and in the appended claims, the singular forms "one," "a," "another," and "said " include the plural designation of the object unless the context clearly indicates otherwise.

As used herein, "buffer" refers to a buffer solution that resists pH changes through the action of its conjugate acid-base pairs. In embodiments of the present invention, a histidine buffer solution is selected, preferably having a pH between about 5.5 and about 6.5, preferably about 6.

As used herein, "surfactant" refers to a surface active agent, and in one embodiment, the surfactant herein is polysorbate 20.

The term "osmolality regulator" refers to a pharmaceutically acceptable osmolality regulator. Suitable osmolality regulators include, but are not limited to, salt, in one embodiment of the present invention sodium chloride (NaCl), at a concentration of about 80 mM to about 160 mM.

The term "stabilizer" refers to a pharmaceutically acceptable stabilizer including, but not limited to, amino acids and sugars, such as arginine hydrochloride, sucrose, and trehalose in embodiments of the present invention, which may be used alone or in combination in concentrations of about 20-80 mM arginine hydrochloride, about 205 mM sucrose, and about 200 mM trehalose.

Protein "stability" can be assessed qualitatively and/or quantitatively after storage at selected temperatures for selected periods in some different ways, including assessment of aggregate formation (e.g. using size exclusion chromatography, measuring turbidity, and/or by visual inspection); assessment of charge heterogeneity using cation exchange chromatography, image capillary isoelectric focusing (icIEF) or capillary zone electrophoresis; analysis of amino-terminal or carboxy-terminal sequence; mass spectrometry; SDS-PAGE analysis to compare reduced and intact antibodies; peptide mapping (e.g., trypsin or LYS-C) analysis; assessment of biological activity or antigen-binding function of antibodies; etc.

In one embodiment of the present invention, the purity of the sample after storage of the selected time period is detected by molecular exclusion high performance liquid chromatography : separation and quantification according to the different molecular sizes, and thus information on the amount of the sample monomers, aggregates, and fragments is obtained; charge isomers are detected by cation exchange high performance liquid chromatography (CEX-HPLC): separation and quantification according to the different protein charges, and thus information on the charge heterogeneity of the sample is obtained; or the charge isomers are detected by imaging capillary isoelectric focusing electrophoresis (IEF): separation and quantification based on the different isoelectric points of the proteins, thus obtaining information on the amount of acidic and basic proteins in the sample; determination of the "biological activity" of the sample by reporter gene assay, which is based on the principle that the binding of PD-L1-expressing target cells to effector cells expressing PD-1 and luciferase inhibits luciferase expression, and the addition of PD-1 antibody blocks the binding of PD-1 to PD-L1, so the biological activity of PD-1 antibody can be determined by analyzing the strength of luciferase expression.

The term "antibody" refers to an immunoglobulin molecule and refers to any form of antibody that exhibits the desired biological activity. These include, but are not limited to, monoclonal antibodies (including full-length monoclonal antibodies), polyclonal antibodies and multi-specific antibodies (e.g., bispecific antibodies), and even antibody fragments. Typically, the full-length antibody structure preferably contains four polypeptide chains, two heavy (H) chains, and two light (L) chains, typically interconnected by disulfide bonds. Each heavy chain contains a heavy chain variable region and a heavy chain constant region. Each light chain contains a light chain variable region and a light chain constant region. In addition to this typical full-length antibody structure, the structure also includes other derivative forms.

The term "variable region" refers to the domain in the heavy or light chain of an antibody that is involved in antibody binding to antigen. The variable regions of the heavy and light chains of natural antibodies (VH and VL, respectively) generally have a similar structure and can be further subdivided into highly variable regions (called complementary determining region (CDR)) interspersed with more conserved regions (called framework regions (FR)).

The term "complementary determining region" (CDR, e.g. CDR1, CDR2, and CDR3) refers to such amino acid residues of the variable region of an antibody whose presence is essential for antigen binding. Each variable region typically has three CDR regions identified as CDR1, CDR2, and CDR3. Each complementary determining region may contain amino acid residues from a "complementary determining region" as defined by Kabat (Kabat et al., Sequences of Proteins of Immunological Interest, 5th Ed. Health Service, National Institutes of Health, Bethesda, MD. 1991) and/or those residues from the "highly variable loop" (Chothia and Lesk; J Mol Biol 196: 901- 917 (1987)).

Each heavy chain variable region and light chain variable region typically contains 3 CDRs and up to 4 FRs, said CDRs and FRs being arranged from the amino terminus to the carboxyl terminus in the following order, for example, FR1, CDR1, FR2, CDR2, FR3, CDR3, FR4.

The complementarity determining region (CDR) and the framework region (FR) of a given antibody can be identified using the Kabat system (Kabat et al: Sequences of Proteins of Immunological Interest, 5th edition, U.S. Department of Health and Human Services, PHS, NIH, NIH Publication No. 91-3242, 1991 ).

The term "constant region" refers to such amino acid sequences in the light and heavy chains of an antibody that is not directly involved in antibody-antigen binding but exhibit a variety of effector functions, such as antibody-dependent cytotoxicity.

An "antigen-binding fragment of an antibody" comprises a portion of an intact antibody molecule that retains at least some of the binding specificity of the parent antibody and typically includes at least a portion of the antigen-binding region or variable region (e.g., one or more CDRs) of the parent antibody. Examples of antigen-binding fragments include, but are not limited to, Fv, Fab, Fab', Fab'-SH, F(ab')2, Fd fragments, Fd' fragments, single-chain antibody molecules (e.g., scFv, di-scFv or tri-scFv, bipartite antibodies or scFab), and single-domain antibodies.

An "antibody fragment" is a non-intact antibody molecule that retains at least some of the biological properties of the parent antibody, including, but not limited to, an Fc fragment, in addition to those described above for "antigen-binding fragments".

The term "chimeric" antibody refers to an antibody in which a portion of the heavy and/or light chain is derived from a specific source or species and the remainder is derived from a different source or species. "Humanized antibodies" are a subset of "chimeric antibodies".

The term "humanized antibody" or "humanized antigen-binding fragment" is defined herein as an antibody or antibody fragment that: (i) antibodies derived from a non-human source (e.g., a transgenic mouse carrying a heterologous immune system) and based on a human germline sequence; or (ii) chimeric antibodies in which the variable region is of non-human origin and the constant region is of human origin; or (iii) CDR grafts in which the CDR in the variable region is of non-human origin and one or more of the framework regions in the variable region is of human origin and the constant region, if any, is of human origin. The purpose of "humanization" is to eliminate the immunogenicity of non-human origin antibodies in humans while retaining the greatest possible affinity. It is advantageous to select the human framework region sequence that is most similar to the framework region sequence of the non-human source antibody as the template for humanization. In some cases, it may be necessary to replace one or more amino acids in the human framework region sequence with the corresponding residues in the non-human framework region to avoid loss of affinity.

The term "monoclonal antibody" refers to an antibody derived from a substantially homogeneous population of antibodies, i.e., every single antibody comprised in the population is identical except for possible mutations (e.g., natural mutations) that may be present in very small amounts. Thus, the term "monoclonal" indicates the nature of said antibodies, i.e., not a mixture of unrelated antibodies. In contrast to polyclonal antibody preparations, which usually include different antibodies against different antigenic determinants (epitopes), each monoclonal antibody of a monoclonal antibody preparation is directed against a separate antigenic determinant. In addition to their specificity, monoclonal antibody preparations have the advantage that they are usually not contaminated with other antibodies. The term "monoclonal" should not be construed as requiring any particular method of producing said antibody. The term monoclonal antibody specifically includes chimeric antibodies, humanized antibodies, and human antibodies.

The antibody "specifically binds" to a target antigen such as a tumor-associated peptide antigen target (herein, PD-1), i.e., binds said antigen with sufficient affinity to allow said antibody to be used as a therapeutic agent, targets a cell or tissue expressing said antigen and does not significantly cross-react with other proteins or with proteins other than the homologs and variants (e.g. mutant forms, splice variants, or proteolytically truncated forms) of the antigenic targets mentioned above.

The term "binding affinity" refers to the strength of the sum of non-covalent interactions between a molecule's individual binding sites and its binding partners. Unless otherwise specified, "binding affinity" as used herein refers to the intrinsic binding affinity, which reflects a 1:1 interaction between members of a binding pair (e.g., antibody and antigen). "KD", "binding rate constant kon" and "dissociation rate constant koff' are commonly used to describe the affinity between a molecule (e.g., antibody) and its binding partner (e.g., antigen), i.e., how tightly a ligand binds a particular protein. The binding affinity is influenced by non-covalent intermolecular interactions such as hydrogen bonding, electrostatic interactions, and hydrophobic and van der Waals forces between two molecules. In addition, the binding affinity between a ligand and its target molecule may be influenced by the presence of other molecules. Affinity can be analyzed by conventional methods known in the art, including the ELISA described herein.

An "isolated" antibody is an antibody that has been identified and isolated from a cell that naturally expresses the antibody. Isolated antibodies include in situ antibodies in recombinant cells and antibodies that are typically prepared by at least one purification step.

The "sequence identity" between two peptide or nucleic acid sequences indicates the number of residues that are identical between said sequences as a percentage of the total number of residues. In calculating the percent identity, the sequences being compared are matched in a manner that produces the maximum match between the sequences, and the empty positions in the match (if present) are resolved by a specific algorithm. Preferred computer program methods for determining identity between two sequences include, but are not limited to, GCG program packages including GAP, BLASTP, BLASTN, and FASTA (Altschul et al. 1990, J. Mol. Biol. 215: 403-410). The above procedures are publicly available from the National Center for Biotechnology Information (NCBI) and other sources. The well-known Smith Waterman algorithm can also be used to determine identity.

One embodiment of the invention employs the recombinant anti-PD-1 monoclonal antibody documented in patent application PCT/CN2019/126594 filed on December 19, 2019, and in a particularly preferred embodiment, the PD1-H944 antibody. PCT/CN2019/126594 is introduced by reference into this specification and the claims.

In a particularly preferred embodiment of the invention, the formulation contains 25 mg/mL PD1-H944 antibody; 20 mM histidine buffer, 120 mM sodium chloride, 40 mM arginine hydrochloride, and 0.02 wt% polysorbate 80. The formulation has good stability and is stable for at least 42 months at 2 to 8°C and at least 12 months at 25°C.

The formulation of the invention may be provided in liquid form or may be provided in lyophilized form. This can be done by reconstituting the lyophilized formulation before administration.

The formulations of the present invention can treat tumors or cancers, preferably colon cancer.

### EXAMPLE

The present invention will be more completely understood by reference to the following embodiments. They should not, however, be construed as limiting the scope of the present invention. All literature, patents, and patent applications are incorporated herein by reference.

In the following embodiments, the recombinant anti-PD-1 monoclonal antibody used is PD1-H944, the preparation, characterization, and performance identification of which is documented in PCT/CN2019/126594, filed on December 19, 2019.

In the following embodiments, the detection method used is as described below.

### (1) Molecular Exclusion High-Performance Liquid Chromatography (SEC-HPLC)

Using a column: TSKgel G3000SWX gel filtration column (7.8×300mm, 5µm) (catalog number 0008541), the mobile phase was SEC mobile phase (200mM disodium hydrogen phosphate, 100mM arginine, pH6.50, 1% isopropanol), UV detection wavelength was 280nm, column temperature was 25°C, 80µg of the test sample was injected into the liquid chromatograph, and the sample purity was calculated according to the area normalization method (General rule 0514 of the Chinese Pharmacopoeia (2015 edition Volume III)).

### 2) Cation exchange high-performance liquid chromatography (CEX-HPLC)

A weak cation exchange column WCX-10 (4^{∗}250 mm) (Product No. 054993) was used with phase A (10 mM PB, pH=7.0) and phase B (10 mM PB, 200 mM NaCl, pH=7.0) as mobile phases, UV detection wavelength was 280 nm, and the column temperature was 35 °C. The gradient elution of SCT-I10A was performed according to the following table.

| | Time (minutes) | B (%) | C(%) | Protective pressure (bar) |
|---|---|---|---|---|
| 1 | 0.00 | 100 | 0 | 200 |
| 2 | 40.00 | 50 | 50 | 200 |
| 3 | 45.00 | 0 | 100 | 200 |
| 4 | 45.01 | 100 | 0 | 200 |
| 5 | 70.00 | 100 | 0 | 200 |

80 µg of the test sample was injected into the liquid chromatograph and the purity of the sample was calculated according to the area normalization method (refer to General rule 0512 and General rule 0513 of the Pharmacopoeia of the People's Republic of China (2015 version volume III)).

### 3) Imaging capillary isoelectric focusing electrophoresis (IEF)

The iCE3 assay was performed using an imaging capillary isoelectric focusing electrophoresis instrument. The relevant parameters for iCIEF were: the sample solution for analysis was prepared by mixing the test sample with Pharmalyte 3-10 for IEF, 1% methylcellulose (1% MC), pI marker with pI of 5.12 and 9.33, and water. The final concentration of protein was 0.25 mg/mL, the final concentration of amphoteric electrolyte was 4%, and the final concentration of MC was 0.35% in the final solution. ICE3 analysis was performed under the following focusing conditions: 1500 V for 1 min; 3000 V for 6 min. After applying voltage to the capillary, the sample will be focused at its pI point. The detection wavelength is 280 nm, and the UV absorption peak is photographed, then the focused spectrum can be obtained, and information on the amount of acidic and basic proteins of the sample can be obtained by calculation (Chinese Pharmacopoeia, 2015 edition, volume IV, General rule 0542 Capillary electrophoresis method).

### 4) Reporting gene method to determine the "biological activity" of the sample

CHO-K1-PD-L1-CD3E cells were inoculated in 96-well plates at 20K/well and incubated overnight at 37°C, 5% CO₂ and then the supernatant was discarded. The cells were added to serial dilutions of working control samples and test samples at 40µL/well to make the final concentrations of 40.000, 13.333, 4.444, 1.481, 0.494, 0.165, 0.055, 0.018, and 0.006 µg/mL, Jurkat-NFAT-Luc2p-PD-1 cells were added at 75K/well (40 µL/well) and incubated for 6 h at 37°C under 5% CO₂ environment, and the cells were lysed and 20 µL/well of cell lysate was taken to a 96-well white bottom plate, placed on a microplate luminescence detector, and 60 µL/well of Luciferase Assay System was added for bioluminescence detection. The relative activity of the samples was calculated by plotting the logarithm of the sample concentration as the horizontal coordinate and the bioluminescence value (RLU) as the vertical coordinate and fitting a four-parameter equation to obtain the EC₅₀ values of the samples and the working control samples. Relative activity (%) = working control sample EC₅₀ / sample EC₅₀ × 100% (Lan Wang, Chuanfei Yu , Yalan Yang, et al. Development of a robust reporter gene assay to measure the bioactivity of anti-PD-1 / anti-PD-L1 therapeutic antibodies. Journal of Pharmaceutical and Biomedical Analysis,2017,145:447-453; Chinese Pharmacopoeia, 2015 Edition Volume IV General 3523 Interferon Biological Activity Assay Method II reporter gene method).

### Example 1: Screening of formulation solution pH

The formulation of PD1-H944 for this embodiment is shown in the following table.

**Table 1 Formulations with different pH**

| Formulation serial number | PD-1 monoclonal antibody | Histidine buffer | Arginine hydrochloride | Sodium chloride | Polysorbate 80 | pH |
|---|---|---|---|---|---|---|
| F1 | 25mg/mL | 40mM | 40mM | 120mM | 0.02wt% | 5.5 |
| F2 | 25mg/mL | 40mM | 40mM | 120mM | 0.02wt% | 5.8 |
| F3 | 25mg/mL | 40mM | 40mM | 120mM | 0.02wt% | 6.0 |
| F4 | 25mg/mL | 40mM | 40mM | 120mM | 0.02wt% | 6.2 |
| F5 | 25mg/mL | 40mM | 40mM | 120mM | 0.02wt% | 6.5 |

Preparation method of antibody formulation: the antibody was exchanged into the target buffer (all components except polysorbate 80 and antibody) by ultrafiltration, supplemented with the required amount of polysorbate 80, and the antibody concentration was adjusted to 25 mg/mL, aseptically dispensed and placed in a 4°C refrigerator and 37°C thermostat, respectively, and taken out at week 0, week 3, and week 5 for analysis and detection, and the assays included SEC -HPLC and IEF.

### Analysis and detection methods:

Purity detection: molecular exclusion high-performance liquid chromatography (SEC-HPLC); its detection principle is to separate and quantify molecules according to their different sizes, and then obtain information on the amount of sample monomers, aggregates, and fragments.

Charge isomers: imaging capillary isoelectric focusing electrophoresis (IEF); its detection principle is to separate and quantify proteins according to their different isoelectric points, and then obtain information on the amount of acidic and basic proteins in the sample; for antibody products, less acidic peaks are appropriate.

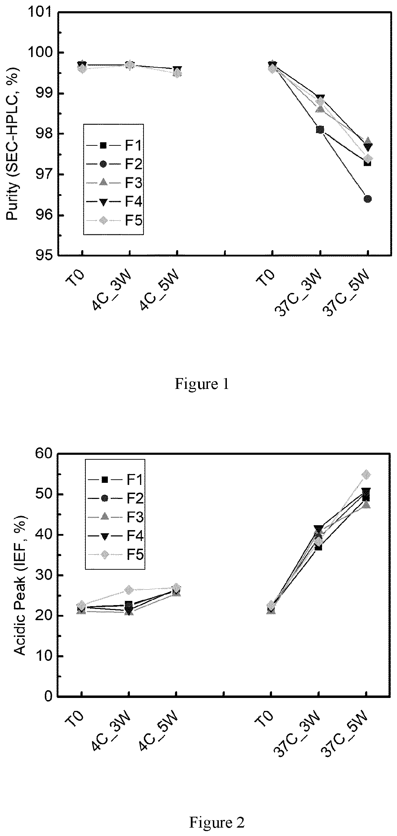

The test results are shown in the attached Figures 1~2.

The test results showed that the purity of PD1-H944 in F3 was higher than in the other formulations and the acidic peak was lower than the other formulations, indicating that the stability of F3 was better than the other formulations.

### Example 2: Screening of antibody concentrations

The formulation of PD1-H944 for this embodiment is shown in the following table.

**Table 2 Formulations with different antibody concentrations**

| Formulation serial number | PD-1 monoclonal antibody | Histidine buffer | Sodium chloride | Polysorbate 80 | pH |
|---|---|---|---|---|---|
| F1 | 25mg/mL | 20mM | 120mM | 0.02wt% | 6.0 |
| F2 | 10mg/mL | 20mM | 120mM | 0.02wt% | 6.0 |

Preparation method of antibody formulation: the antibody was exchanged into the target buffer (all components except polysorbate 80 and antibody) by ultrafiltration, supplemented with the required amount of polysorbate 80, and then the antibody concentration was adjusted to 10 mg/mL and 25 mg/mL, respectively, aseptically dispensed and placed in 4°C refrigerator and 37°C thermostat, and taken our at week 0, week 3 and week 5 for analysis, respectively The assays included SEC-HPLC and IEF.

### Analysis and detection methods:

Purity testing: molecular exclusion high-performance liquid chromatography.

Charge isomers: imaging capillary isoelectric focusing electrophoresis.

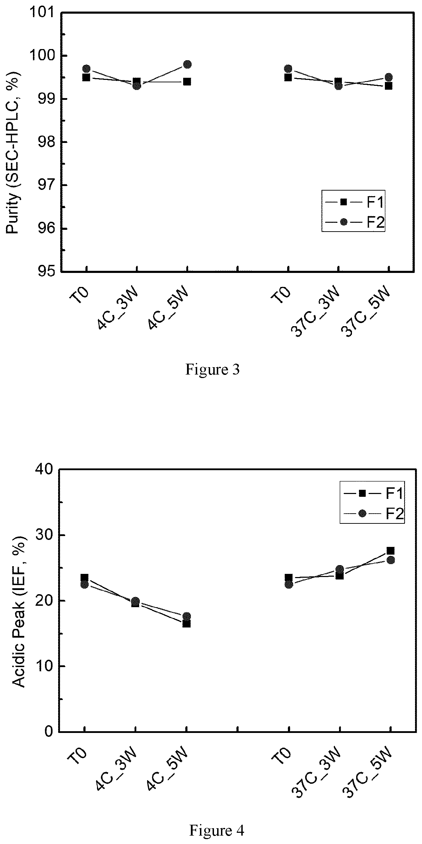

The test results are shown in the attached Figures 3-4.

The assay results showed that the purity of PD1-H944 in F1 and F2 was higher than 99% when placed at 37°C for 0, 3 and 5 weeks and the changing trend of the acidic peak was basically the same, indicating that the stability of PD1-H944 in F1 and F2 was comparable.

### Example 3: Screening of the concentration of surfactants

The formulation of PD1-H944 of this embodiment is shown below.

**Table 3 Formulations with different surfactant concentrations**

| Formulation serial number | PD-1 monoclonal antibody | Histidine buffer | Sodium chloride | Polysorbate 80 | pH |
|---|---|---|---|---|---|
| F1 | 25mg/mL | 20mM | 120mM | 0.02wt% | 6.0 |
| F2 | 25mg/mL | 20mM | 120mM | 0.04wt% | 6.0 |

Preparation method of antibody formulation: the antibody was exchanged into the target buffer (all components except polysorbate 80 and antibody) by ultrafiltration, supplemented with the required amount of polysorbate 80, and the antibody concentration was adjusted to 25 mg/mL, aseptically dispensed and placed in a 4°C refrigerator and 37°C thermostat, respectively, and taken out at week 0, week 3, and week 5 for analysis and detection, and the assays included SEC -HPLC and IEF.

### Analysis and detection methods:

Purity testing: molecular exclusion high performance liquid chromatography.

Charge isomers: imaging capillary isoelectric focusing electrophoresis.

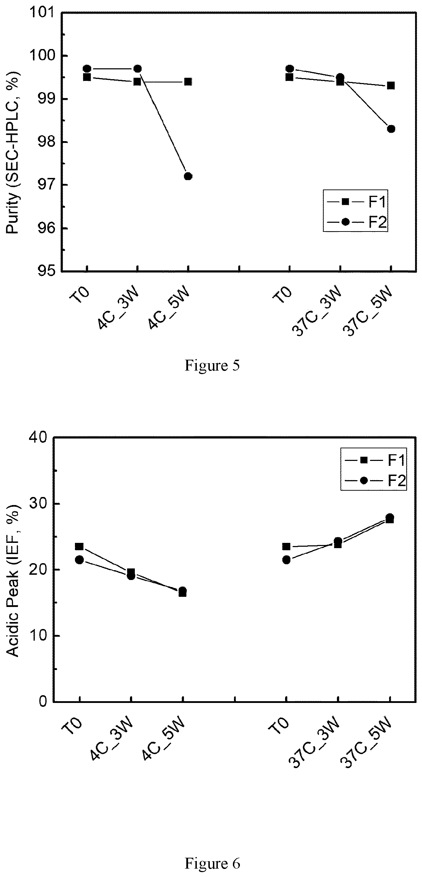

The test results are shown in the attached Figures 5~6.

The detection results showed that the purity of PD1-H944 in F1 was higher than that of F2, and the trend of the acidic peak change was basically the same, indicating that the stability of F1 was better than that of F2.

### Example 4: Screening of concentrations of osmolarity regulators

The formulation of PD1-H944 of this embodiment is shown below.

**Table 4 Formulations with different osmolality regulator concentrations**

| Formulation serial number | PD-1 monoclonal antibody | Histidine buffer | Sodium chloride | Polysorbate 80 | pH |
|---|---|---|---|---|---|
| F1 | 25mg/mL | 20mM | 120mM | 0.02wt% | 6.0 |
| F2 | 25mg/mL | 20mM | 160mM | 0.02wt% | 6.0 |
| F3 | 25mg/mL | 20mM | 80mM | 0.02wt% | 6.0 |

Preparation method of antibody formulation: the antibody was exchanged into the target buffer (all components except polysorbate 80 and antibody) by ultrafiltration, supplemented with the required amount of polysorbate 80, and the antibody concentration was adjusted to 25 mg/mL, aseptically dispensed and placed in 4°C refrigerator and 37°C thermostat respectively, and taken out for analysis and detection at week 0, week 3 and week 5 respectively.

### Analysis and detection methods:

Purity test: molecular exclusion high-performance liquid chromatography.

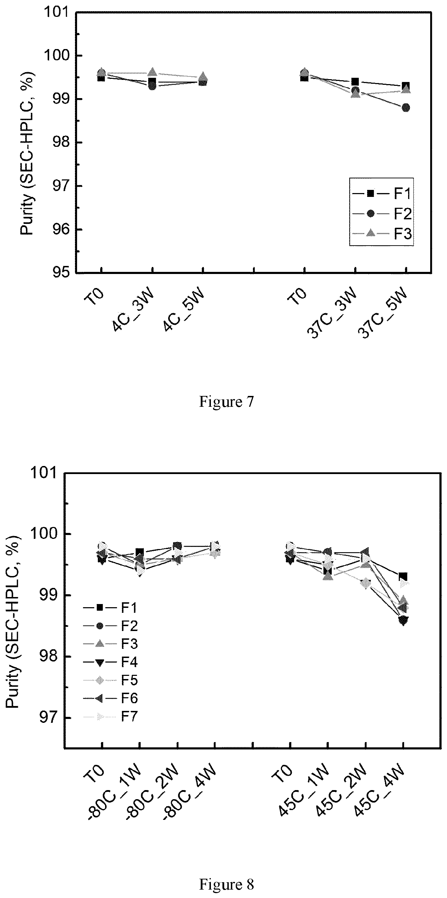

The test results are shown in the attached Figure 7.

The assay results showed that the purity of PD1-H944 in F1 was higher than that of F2 and F3, indicating that the stability of F1 was better than that of F2 and F3.

### Example 5: Screening of stabilizer and stabilizer concentration

The formulation of PD1-H944 of this embodiment is shown below.

**Table 5 Formulations with different stabilizers and different stabilizer concentrations***

| Formulation serial number | PD-1 monoclonal antibody | Histidine buffer | Sodium chloride | Sucrose | Arginine hydrochloride | Trehalose | Polysorbate 80 |
|---|---|---|---|---|---|---|---|
| F1 | 25mg/mL | 20mM | 120mM | | 40mM | | 0.02wt% |
| F2 | 25mg/mL | 20mM | 120mM | | 80 mM | | 0.02wt% |
| F3 | 25mg/mL | 20mM | 120mM | | 20 mM | | 0.02wt% |
| F4 | 25mg/mL | 20mM | | 205mM | | | 0.02wt% |
| F5 | 25mg/mL | 20mM | | | | 200mM | 0.02wt% |
| F6 | 25mg/mL | 20mM | | 205mM | 40mM | | 0.02wt% |
| F7 | 25mg/mL | 20mM | | | 40mM | 200mM | 0.02wt% |

| | | | | | | | |
|---|---|---|---|---|---|---|---|
| ^{∗} pH 6.0 in each formulation | | | | | | | |

Preparation method of antibody formulation: the antibody was exchanged into the target buffer (all components except polysorbate 80 and antibody) by ultrafiltration, supplemented with the required amount of polysorbate 80, and the antibody concentration was adjusted to 25 mg/mL, aseptically dispensed and placed in -80°C refrigerator and 45°C thermostat, and taken out for analysis and detection at week 0, week 1, week 2 and week 4, respectively.

### Analysis and detection methods:

Purity test: molecular exclusion high-performance liquid chromatography.

The test results are shown in the attached Figure 8.

The test results showed that the purity of PD1-H944 in F1 was higher than in the other formulations at 45°C for 4 weeks, indicating that the stability of F1 was better than the other formulations.

### Example 6: Formulation validation assay

Three batches of recombinant PD-1 monoclonal antibody formulations (formula of 25 mg/mLPD1-H944 + 20 mM histidine buffer + 120 mM sodium chloride + 40 mM arginine hydrochloride + 0.02 wt% polysorbate 80, pH 6.0) were assayed for stability at 2~8°C and 25±2°C for SEC-HPLC, CEX-HPLC and Biological activity.

### Analysis and detection methods:

Purity testing: molecular exclusion high-performance liquid chromatography.

Charge isomers: Cation exchange high-performance liquid chromatography (CEX-HPLC); its detection principle is to separate and quantify proteins according to their different charges, and thus obtain information on the charge heterogeneity of the sample.

Biological activity: reporter gene method; the principle of the assay is that the binding of PD-L1-expressing target cells to effector cells expressing PD-1 and luciferase inhibits the expression of luciferase, and the addition of PD-1 antibody blocks the binding of PD-1 to PD-L1, so the biological activity of PD-1 antibody can be determined by analyzing the strength of luciferase expression (see PCT/CN2019 /126594).

The experimental results are shown in Tables 6 to 11.

**Table 6 Stability data of PD1-H944 formulation (Lot1) stored at 4°C**

| Inspection Items | | Storage period at 4°C(months) | | | | | | | | | |
|---|---|---|---|---|---|---|---|---|---|---|---|
| | | 0 | 3 | 6 | 9 | 12 | 18 | 24 | 30 | 36 | 42 |
| Purity (SEC-HPLC, %) | Monomers | 99.5 | 99.4 | 99.4 | 99.0 | 99.1 | 99.3 | 99.2 | 99.2 | 99.1 | 99.4 |
| | Aggregates | 0.5 | 0.6 | 0.6 | 1.0 | 0.9 | 0.7 | 0.7 | 0.8 | 0.9 | 0.6 |
| | Fragments | 0.0 | 0.0 | 0.0 | 0.0 | 0.0 | 0.0 | 0.0 | 0.0 | 0.0 | 0.0 |
| Charge isomers (CEX-HPLC, %) | Acidic peaks | 12.3 | 12.0 | 13.2 | 13.8 | 14.5 | 15.6 | 15.7 | 16.9 | 18.2 | 18.9 |
| | Lysine variants | 87.7 | 88.1 | 86.8 | 86.1 | 85.5 | 84.4 | 84.2 | 83.2 | 81.8 | 81.0 |
| Biological activity (%) | | 100 | 93 | 92 | 87 | 97 | 86 | 94 | 88 | 87 | 83 |

**Table 7 Stability data of PD1-H944 formulation (Lot1) stored at 25°C**

| Inspection Items | | Storage period at 25°C(months) | | | | | | |
|---|---|---|---|---|---|---|---|---|
| | | 0 | 2 | 4 | 6 | 8 | 10 | 12 |
| Purity(SEC-HPLC,%) | Monomers | 99.5 | 99.3 | 99.2 | 99.1 | 99.0 | 98.1 | 96.8 |
| | Aggregates | 0.5 | 0.7 | 0.7 | 0.8 | 0.9 | 1.8 | 3.0 |
| | Fragments | 0.0 | 0.0 | 0.1 | 0.1 | 0.1 | 0.1 | 0.2 |
| Charge isomers(CEX-HPLC,%) | Acidic peaks | 12.3 | 17.2 | 20.3 | 25.3 | 29.7 | 33.7 | 39.4 |
| | Lysine variants | 87.7 | 82.8 | 79.7 | 74.8 | 70.3 | 66.3 | 60.6 |
| Biological activity(%) | | 100 | 92 | 93 | 90 | 88 | 81 | 90 |

**Table 8 Stability data of PD1-H944 formulation (Lot2) stored at 4°C**

| Inspection Items | | Storage period at 4°C(months) | | | | | | | | | |
|---|---|---|---|---|---|---|---|---|---|---|---|
| | | 0 | 3 | 6 | 9 | 12 | 18 | 24 | 30 | 36 | 42 |
| Purity(SEC-HPLC,%) | Monomers | 99.6 | 99.5 | 99.4 | 99.1 | 98.9 | 99.3 | 99.2 | 99.2 | 99.2 | 99.4 |
| | Aggregates | 0.4 | 0.5 | 0.6 | 0.9 | 1.1 | 0.7 | 0.8 | 0.8 | 0.8 | 0.6 |
| | Fragments | 0.0 | 0.0 | 0.0 | 0.0 | 0.0 | 0.0 | 0.0 | 0.0 | 0.0 | 0.0 |
| Charge isomers(CEX- | Acidic peaks | 13.6 | 13.4 | 14.4 | 15.3 | 14.7 | 16.7 | 16.8 | 18.0 | 18.5 | 19.6 |
| HPLC,%) | Lysine variants | 86.5 | 86.6 | 85.6 | 84.7 | 85.3 | 83.2 | 83.1 | 82.0 | 81.6 | 80.4 |
| Biological activity(%) | | 92 | 99 | 93 | 97 | 95 | 104 | 75 | 84 | 78 | 86 |

**Table 9 Stability data of PD1-H944 formulation (Lot2) stored at 25°C**

| Inspection Items | | Storage period at 25°C(months) | | | | | | |
|---|---|---|---|---|---|---|---|---|
| | | 0 | 2 | 4 | 6 | 8 | 10 | 12 |
| Purity(SEC-HPLC,%) | Monomers | 99.6 | 99.4 | 99.1 | 98.9 | 99.0 | 98.5 | 98.3 |
| | Aggregates | 0.4 | 0.6 | 0.8 | 1.0 | 1.0 | 1.4 | 1.6 |
| | Fragments | 0.0 | 0.0 | 0.1 | 0.1 | 0.1 | 0.1 | 0.1 |
| Charge isomers(CEX-HPLC,%) | Acidic peaks | 13.6 | 18.0 | 22.0 | 27.1 | 31.5 | 32.8 | 38.4 |
| | Lysine variants | 86.5 | 82.0 | 78.0 | 72.9 | 68.6 | 67.2 | 61.6 |
| Biological activity(%) | | 92 | 87 | 92 | 97 | 87 | 74 | 90 |

**Table 10 Stability data of PD1-H944 formulation (Lot3) stored at 4°C**

| Inspection Items | | Storage period at 4°C(months) | | | | | | | | | |
|---|---|---|---|---|---|---|---|---|---|---|---|
| | | 0 | 3 | 6 | 9 | 12 | 18 | 24 | 30 | 36 | 42 |
| Purity(SEC-HPLC,%) | Monomers | 99.6 | 99.5 | 99.5 | 99.2 | 98.9 | 99.3 | 99.4 | 99.3 | 99.2 | 99.5 |
| | Aggregates | 0.4 | 0.5 | 0.5 | 0.8 | 1.1 | 0.7 | 0.6 | 0.7 | 0.8 | 0.5 |
| | Fragments | 0.0 | 0.0 | 0.0 | 0.0 | 0.0 | 0.0 | 0.0 | 0.0 | 0.0 | 0.0 |
| Charge isomers(CEX-HPLC,%) | Acidic peaks | 13.6 | 13.6 | 14.9 | 15.6 | 15.7 | 16.6 | 17.2 | 17.7 | 19.5 | 19.5 |
| | Lysine variants | 86.4 | 86.4 | 85.1 | 84.5 | 84.3 | 83.4 | 82.8 | 82.3 | 80.5 | 80.4 |
| Biological activity(%) | | 100 | 100 | 100 | 104 | 86 | 100 | 82 | 90 | 96 | 90 |

**Table 11 Stability data of PD1-H944 formulation (Lot3) stored at 25°C**

| Inspection Items | | Storage period at 25°C(months) | | | | | | |
|---|---|---|---|---|---|---|---|---|
| | | 0 | 2 | 4 | 6 | 8 | 10 | 12 |
| Purity(SEC-HPLC,%) | Monomers | 99.6 | 99.4 | 99.2 | 99.1 | 99.0 | 98.5 | 98.6 |
| | Aggregates | 0.4 | 0.6 | 0.7 | 0.9 | 0.9 | 1.4 | 1.3 |
| | Fragments | 0.0 | 0.0 | 0.0 | 0.1 | 0.1 | 0.1 | 0.1 |
| Charge isomers(CEX-HPLC,%) | Acidic peaks | 13.6 | 19.1 | 23.7 | 27.6 | 32.2 | 33.8 | 38.9 |
| | Lysine variants | 86.4 | 80.9 | 76.3 | 72.3 | 67.8 | 66.3 | 61.1 |
| Biological activity(%) | | 100 | 93 | 92 | 95 | 94 | 82 | 94 |

The experimental results show that the PD1-H944 formulation disclosed in the present invention has good stability and can be stored stably for at least 42 months at 2~8°C and at least 12 months at 25°C.

### SEQUENCE LISTING

| Sequence number | Sequence description | Amino acid sequence |
|---|---|---|
| SEQ ID NO:1 | The amino acid sequence of light chain CDR1 of Mouse antibody PD1-M944 / humanized antibody PD1-H944 | ESVDSYGNSFMH |
| SEQ ID NO:2 | The amino acid sequence of light chain CDR2 of Mouse antibody PD1-M944 / humanized antibody PD1-H944 | AASNQGSGVPA |
| SEQ ID NO:3 | The amino acid sequence of light chain CDR3 of Mouse antibody PD1-M944 / humanized antibody PD1-H944 | QQSKEVPWT |
| SEQ ID NO:4 | The amino acid sequence of heavy chain CDR1 of Mouse antibody PD1-M944 / humanized antibody PD1-H944 | GFTFSSYGMS |
| SEQ ID NO:5 | The amino acid sequence of heavy chain CDR2 of Mouse antibody PD1-M944 / humanized antibody PD1-H944 | VATISGGGRDTYYSDSVKG |
| SEQ ID NO:6 | The amino acid sequence of heavy chain CDR3 of Mouse antibody PD1-M944 / humanized antibody PD1-H944 | SRQYGTVWFFN |
| SEQ ID NO:7 | The amino acid sequence of humanized antibody PD1-H944 heavy chain variable region | EVQLVESGGGLVKPGGSLRLSCAASGFTFS SYGMSWVRQAPGKRLEWVATISGGGRDT YYSDSVKGRFTISRDNAKNNLYLQMNSLR AEDTAVYYCSRQYGTVWFFNWGQGTLVT VSS |
| SEQ ID NO:8 | The amino acid sequence of humanized antibody PD1-H944 light chain variable region | EIVLTQSPATLSLSPGERATLSCRASESVDS YGNSFMHWYQQKPGQPPRLLIYAASNQGS GVPARFSGSGSGTDFTLTISSLEPEDFAMYF CQQSKEVPWTFGQGTKVEIK |
| SEQ ID NO:9 | The amino acid sequence of humanized antibody PD1-H944 heavy chain constant region | ASTKGPSVFPLAPCSRSTSESTAALGCLVK DYFPEPVTVSWNSGALTSGVHTFPAVLQSS GLYSLSSVVTVPSSSLGTKTYTCNVDHKPS NTKVDKRVESKYGPPCPPCPAPEFLGGPSV FLFPPKPKDTLMISRTPEVTCVVVDVSQED PEVQFNWYVDGVEVHNAKTKPREEQFNS TYRVVSVLTVLHQDWLNGKEYKCKVSNK GLPSSIEKTISKAKGQPREPQVYTLPPSQEE MTKNQVSLTCLVKGFYPSDIAVEWESNGQ PENNYKTTPPVLDSDGSFFLYSRLTVDKSR WQEGNVFSCSVMHEALHNHYTQKSLSLS LGK |
| SEQ ID NO: 10 | The amino acid sequence of humanized antibody PD1-H944 light chain constant region | |

## Claims

1. A stable formulation comprising
a recombinant anti-PD-1 monoclonal antibody of 10-50 mg/mL, preferably a recombinant anti-PD-1 monoclonal antibody of 10-25 mg/mL;
a buffer of 10-50 mM, preferably a buffer of 20-40 mM;
an osmolality regulator of 20-200 mM, preferably an osmolality regulator of 80-160 mM;
a stabilizer of 10-250 mM, preferably a stabilizer of 20-205 mM;
a surfactant of 0.005-0.05 wt%, preferably a surfactant of 0.02-0.04 wt%;
the pH of the solution is 5.5-6.5, preferably 5.8-6.2.

2. The formulation according to claim 1, wherein
said buffer is selected from one or more of citric acid buffer, acetate buffer, or histidine buffer; said osmolality regulator is selected from sodium chloride;
said stabilizer is one or more of sucrose, trehalose, or arginine hydrochloride, preferably 205 mM sucrose, or 20 mM - 80 mM arginine hydrochloride, or 200 mM trehalose;
said surfactant is selected from polysorbate 80.

3. The formulation according to any one of claims 1-2, wherein the formulation solution has a pH of 6.0.

4. The formulation according to any one of claims 1-3, comprising 25 mg/mL recombinant anti-PD-1 monoclonal antibody; 20 mM histidine buffer, 120 mM sodium chloride, 40 mM arginine hydrochloride, and 0.02 wt% polysorbate 80.

5. The formulation according to any one of claims 1-4, wherein said recombinant anti-PD-1 monoclonal antibody comprises a light chain variable region and/or a heavy chain variable region,
wherein the light chain variable region comprises a light chain CDR1 with amino acid sequence SEQ ID NO: 1, a light chain CDR2 with amino acid sequence SEQ ID NO:2 and a light chain CDR3 with amino acid sequence SEQ ID NO:3;
the heavy chain variable region comprises heavy chain CDR1 with amino acid sequence SEQ ID NO:4, heavy chain CDR2 with amino acid sequence SEQ ID NO:5, and heavy chain CDR3 with amino acid sequence SEQ ID NO:6.

6. The formulation according to claim 5, wherein said recombinant anti-PD-1 monoclonal antibody comprises an amino acid sequence having at least 90%, 92%, 95%, 98%, or 100% sequence identity to the PD-1 antibody light chain variable region sequence SEQ ID NO:8, and/or an amino acid sequence having at least 90%, 92%, 95%, 98% or 100% sequence identity to the PD-1 antibody heavy chain variable region sequence SEQ ID NO:7.

7. The formulation according to claim 5 or 6, wherein said antibody further comprises a light chain constant region and a heavy chain constant region, preferably the amino acid sequence of said light chain constant region having at least 90%, 92%, 95%, 98%, or 100% sequence identity to the kappa light chain constant region of SEQ ID NO: 10, and/or the amino acid sequence of said heavy chain constant region having at least 90%, 92%, 95%, 98% or 100% sequence identity to the IgG4 heavy chain constant region of SEQ ID NO:9.

8. The formulation according to claim 7, which is an IgG antibody, preferably an IgG4 antibody.

9. The formulation according to claim 7 or 8, which is a monoclonal antibody.

10. The formulation according to any one of claims 7-9, having a mean KD, which binds to recombinant human PD-1 protein with an affinity in KD average of 20-200 pM, preferably 60-70 pM, more preferably 64.8 pM.

11. The formulation according to any one of claims 1-10, which is in the form of an aqueous formulation or lyophilized form.

12. The use of a formulation according to any one of claims 1-11 in the preparation of a medicament for the treatment of a tumor or cancer, preferably colon cancer.

13. The formulation according to any one of claims 1-11 for use in the treatment of a tumor or cancer, preferably colon cancer.

14. The formulation according to any of claims 1-11, which can be stored stably for at least 42 months at 2 to 8°C and for at least 12 months at 25°C.
